# FASCICULE DE BREVET EUROPEEN

(11) **EP 1 903 032 B1**
(45) Date de publication et mention de la délivrance du brevet: **14.08.2013**
(21) Numéro de dépôt: 07023115.4
(22) Date de dépôt: 28.05.2001
(51) Int. Cl.: C07D 201/08, C07D 201/16

(54) **Procédé de purification de lactames**
Verfahren zur Reinigung von Lactamen
Method for purifying lactams

(30) Priorité: 26.05.2000 FR 0006784
(43) Date de publication de la demande: 26.03.2008
(62) Demande divisionnaire de: 01940616.4
(73) Titulaire: Rhodia Opérations, 93306 Aubervilliers (FR)
(72) Inventeur: Amoros, Daniel, 69200 Venissieux (FR); Leconte, Philippe, 68150 Ribeauvillé (FR); Coqueret, Pierre, 69340 Francheville (FR)
(74) Mandataire: Trolliet, Maurice

(56) Documents cités:
- EP-A- 0 306 874
- WO-A-98/05636
- DD-A- 81 846
- DD-A- 152 914
- DD-A- 226 560
- DD-A- 226 561
- DD-A- 226 562
- DD-A- 267 729
- DE-A- 3 106 350
- DE-A- 19 548 289
- US-A- 4 301 073
- US-A- 4 892 624
- US-A- 5 136 052

## Description

La présente invention concerne un procédé de purification de lactames, plus particulièrement de lactames obtenus par hydrolyse cyclisante d'aminonitrile.

L'invention se rapporte plus particulièrement à la purification de l'ε-caprolactame obtenu par hydrolyse cyclisante de l'aminocapronitrile.

Plusieurs procédés de synthèse de l'ε-caprolactame, un grand intermédiaire chimique utilisé notamment dans la production des polyamides et plus particulièrement du polyamide-6, ont été proposés. Un des procédés les plus utilisés industriellement consiste en un réarrangement de Beckmann de l'oxime de cyclohexanone en présence d'acide sulfurique ou d'oléum.

Un autre procédé qui fait l'objet de nombreux travaux, consiste à obtenir l'ε-caprolactame par hydrolyse cyclisante de l'aminocapronitrile. Cette réaction décrite dans le brevet français 2 029 540 peut être réalisée en phase liquide en présence de solvants et de catalyseurs ou comme décrit dans le brevet US 2,357,484, en phase gazeuse avec comme catalyseur de l'alumine.

Le brevet Ep 150295 concerne également un procédé d'hydrolyse cyclisante de l'aminocapronitrile en phase gazeuse en présence d'un catalyseur à base de cuivre et de vanadium.

L'ε-caprolactame obtenu par ces procédés de synthèse doit être purifié pour obtenir un intermédiaire chimique ou monomère permettant notamment la fabrication de polymères présentant des caractéristiques satisfaisantes à la fabrication de produits finis tels que fils, fibres etc ....

De nombreux procédés de purification ont été proposés pour le traitement du caprolactame obtenu par réarrangement de Beckmann de l'oxime de cyclohexanone.

Il a également été proposé des procédés de purification du caprolactame obtenu par hydrolyse cyclisante de l'aminocapronitrile. Ainsi, le brevet US 5,496,941 décrit un procédé consistant à séparer les produits à points d'ébullition bas et les produits à points d'ébullition élevés du milieu de réaction d'hydrolyse. Cette séparation est obtenue notamment par distillation du caprolactame.

Le caprolactame ainsi récupéré est soumis à une hydrogénation puis à soit un traitement sur résines échangeuses d'ions soit une distillation en présence d'acide sulfurique et enfin à une distillation en présence d'une base.

D'autres procédés de purification ont été proposés. Ainsi, le brevet WO 98/05636 propose un procédé de purification par extraction liquide/liquide à l'aide d'un solvant à caractère acide ou par un traitement sur résines.

Dans ces procédés, il est généralement prévu une distillation finale du caprolactame pour obtenir un produit satisfaisant aux critères de pureté requis notamment pour son utilisation comme monomère dans la fabrication des polyamides tels que le polyamide 6.

Toutefois, cette distillation finale est génératrice de perte élevée en caprolactame qui nuit fortement à l'économie générale du procédé.

Dans le procédé décrit dans le document WO98/05636, le caprolactame est distillé dans une seule colonne, le caprolactame de pureté élevée étant collecté en tête de colonne. Toutefois, dans une telle colonne, la fraction de queue contient beaucoup de caprolactame car il est impossible d'épuiser cette fraction de queue en caprolactame tout en récupérant en tête un caprolactame pur. Dans une telle installation, la quantité de caprolactame récupéré est relativement faible (environ 70%).

Ainsi, dans le cas de la purification d'un caprolactame obtenu par la réaction de réarrangement Beckmann, le brevet US 4,301,073 propose un procédé pour purifier le caprolactame comprenant une étape d'extraction par un solvant puis une distillation en milieu basique. Le résidu de distillation est traité pour récupérer le maximum de caprolactame. Ce traitement consiste en de nouvelles distillations et un traitement par un acide fort du caprolactame ainsi distillé avant son recyclage au niveau de l'étape d'extraction. Ce procédé permet d'améliorer le taux de récupération du caprolactame mais requiert l'utilisation d'un acide pour traitement avant recyclage. Ce procédé de recyclage est complexe et demande des investissements importants pour effectuer le traitement d'acide. De plus, ce procédé ne peut être transposé au traitement d'un caprolactame synthétisé par une nouvelle réaction qui peut générer des impuretés totalement différentes requérant de nouveaux procédés de purification.

Un des buts de la présente invention est de remédier à ces inconvénients en proposant un procédé et une installation de purification d'un lactame issu de l'hydrolyse cyclisante d'un aminonitrile permettant d'obtenir un fort taux de récupération de lactame à pureté élevée.

A cet effet, l'invention propose un procédé de purification de lactame, plus particulièrement d'ε-caprolactame, obtenu par hydrolyse cyclisante d'un aminonitrile, plus particulièrement de l'aminocapronitrile, consistant à éliminer l'ammoniac du milieu réactionnel d'hydrolyse puis à récupérer le lactame dudit milieu sous forme purifiée.

Selon l'invention, cette récupération est réalisée en mettant en oeuvre au moins une distillation du lactame en présence d'une base produisant éventuellement une fraction de tête comprenant des composés plus volatils que le lactame, une fraction comprenant le lactame à récupérer au degré de pureté désiré et un pied de distillation comprenant du lactame et des composés moins volatils que le lactame.

Ainsi, le pied ou résidu de distillation récupéré dans l'étape de purification par distillation comprend majoritairement du lactame, les impuretés étant constitués principalement de sels et de sous-produits de réaction.

Ce pied de distillation est soumis, selon l'invention, à un traitement consistant à séparer la majeure partie du lactame contenu dans le pied de distillation et à recycler ledit lactame séparé dans une étape quelconque du procédé de purification ou dans le milieu obtenu après l'hydrolyse cyclisante.

Selon l'invention, ce traitement du pied de distillation consiste soit en une récupération du lactame par, par exemple, évaporation en couches minces du lactame contenu dans le pied de distillation et à recycler ledit lactame séparé dans une étape quelconque du procédé de purification ou dans le milieu obtenu après l'hydrolyse cyclisante.

Selon l'invention, ce traitement du pied de distillation consiste soit en une récupération du lactame par, par exemple, évaporation en couches minces du lactame ou cristallisation de celui-ci soit en une séparation du lactame des autres impuretés, par exemple, par extraction de la majeure partie des impuretés par de l'eau.

La cristallisation du lactame peut être réalisée selon les procédés classiques de cristallisation, avec comme solvant soit de l'eau, soit une solution aqueuse saturée en lactame ou le lactame lui-même. Elle est avantageusement réalisée en une seule étape. Toutefois, si cela est nécessaire, deux ou plusieurs étapes de cristallisation peuvent être mis en oeuvre.

Un procédé de cristallisation et les conditions pour sa mise en oeuvre sont décrits, par exemple, dans les brevets Ep 943608 et US 4,882,430.

Le lactame ainsi obtenu est recyclé dans le dispositif de distillation en milieu basique, soit par alimentation directe dans la colonne de distillation à un niveau déterminé, soit par addition dans le flux de milieu réactionnel entrant dans ladite colonne, soit à un autre stade du procédé de purification et récupération du lactame ou même dans le milieu issu de l'hydrolyse cyclisante.

Selon un deuxième mode de réalisation de l'invention, le pied de distillation est traité dans un procédé d'évaporation en film ou couche mince. Le lactame vaporisé est recyclé dans le dispositif de distillation dans des conditions similaires à celles du mode de réalisation mettant en oeuvre la cristallisation. L'évaporation du lactame est obtenue par chauffage à une température comprise entre 130°C et 150°C, sous une pression inférieure à 10 mbar.

Enfin, dans un troisième mode de réalisation le pied de distillation peut être traité par de l'eau pour dissoudre les impuretés, notamment les sels, le lactame récupéré après décantation étant avantageusement recyclé dans le procédé de purification du lactame par distillation en milieu acide puis milieu basique.

Ces procédés de traitement du pied de distillation permettent de récupérer au moins 90 % en poids, avantageusement au moins 95 % du lactame contenu dans ledit pied. Les pertes de lactame du procédé de purification sont ainsi limitées à moins de 3 % en poids du lactame engagé.

Le procédé de l'invention permet donc de limiter les pertes en lactame à un niveau très faible lors de la purification par distillation basique. Ces pertes affectent que très faiblement l'économie du procédé et surtout la qualité du lactame récupéré est d'un niveau très élevé.

Selon l'invention, le milieu contenant le lactame à récupérer peut être le milieu réactionnel de l'hydrolyse cyclisante d'un aminonitrile après évaporation de l'ammoniac. Ledit milieu réactionnel a pu être soumis à une hydrogénation avant l'évaporation de l'ammoniac.

Ce milieu réactionnel entrant dans la distillation basique peut avoir subi plusieurs traitements préalables après évaporation de l'ammoniac.

Dans un mode de réalisation de l'invention, le milieu réactionnel d'hydrolyse est traité pour éliminer les produits respectivement moins volatils et plus volatils que le lactame. Ce traitement décrit dans le brevet US 5,496,941 consiste à évaporer l'ammoniac, l'eau et les autres produits volatils, puis à distiller le lactame. Le milieu réactionnel d'hydrolyse traité par la distillation basique de la présente invention sera donc le lactame récupéré par distillation.

Dans un autre mode de réalisation, notamment quand l'hydrolyse cyclisante est mise en oeuvre en phase vapeur, uniquement l'ammoniac est extrait du milieu réactionnel ayant subi ou non une hydrogénation préalablement à l'élimination de l'ammoniac.

Le lactame récupéré par distillation ou le milieu réactionnel sans ammoniac peut être soumis à une hydrogénation, un traitement oxydant, par exemple, avant d'être alimenté dans la distillation basique.

Dans un autre mode de réalisation, le procédé de purification du lactame peut comprendre un traitement sur résine échangeuse d'ions en milieu acide ou une distillation en présence d'un acide fort par exemple l'acide sulfurique.

Le procédé de purification et de récupération du lactame peut également comprendre une ou plusieurs cristallisations successives du lactame, le lactame ainsi cristallisé formant le milieu alimenté dans la distillation basique conforme à l'invention.

En conséquence, le procédé de distillation conforme à l'invention est une étape d'un procédé de purification de lactame pouvant être combinée avec de nombreux autres traitements connus des lactames et utilisés dans les nombreux procédés de purification décrits.

Selon l'invention, le procédé de purification de l'invention peut être mis en oeuvre dans un procédé de distillation comprenant plusieurs colonnes de distillation montées en série pour réaliser la séparation successive des différents produits.

Selon le procédé de l'invention, le lactame à purifier est alimenté dans une première colonne de distillation dans laquelle est alimenté. Dans cette colonne, appelée colonne de déshydratation permettant d'éliminer la majorité, de préférence la totalité de l'eau sous forme de fraction de tête, le pied de distillation contenant le lactame étant alimenté dans une seconde colonne de distillation.

Dans la seconde colonne, les composés les moins volatiles, et notamment moins volatiles que le lactame sont séparés et constituent un pied de distillation, le lactame récupéré en tête de colonne est alimenté dans la troisième colonne.

Dans cette dernière colonne, le lactame purifié est recueilli sous forme, avantageusement, de fraction intermédiaire par soutirage soit en phase vapeur, soit en phase liquide. La fraction de tête constituée par les composés plus volatiles que le lactame est éliminé, le pied de distillation est de préférence recyclé dans la seconde colonne.

Dans ce mode de réalisation, le pied de distillation de la seconde colonne est avantageusement traité pour récupérer le lactame selon le procédé de l'invention. Le lactame ainsi récupéré est recyclé avantageusement dans la seconde colonne.

De manière préférée, un composé basique est ajouté au milieu à traiter avant son alimentation dans la première étape de déshydratation.

Toutefois, l'invention concerne également un procédé consistant à traiter le milieu contenant le lactame à purifier par une distillation en milieu acide et plus particulièrement en milieu acide sulfurique suivie d'une distillation en milieu basique.

Dans ce mode de réalisation, l'acide est ajouté au milieu à traiter avant son alimentation dans la colonne de déshydratation, la base étant ajoutée à la fraction de tête produite dans la seconde colonne de distillation avant son alimentation dans la troisième colonne de distillation.

Les sels générés par la présence de l'acide, par exemple, les sulfates d'amines, les sulfates provenant de la neutralisation de la base sont éliminés et purgés, dans le pied de distillation produit à la seconde colonne de distillation. Ce pied de distillation est avantageusement soumis à un traitement de récupération de lactame qui, dans le cas présent, pourra avantageusement être :
- soit, un lavage du pied de distillation par de l'eau pour dissoudre les sels tels que les sulfates d'ammonium et/ou d'amines, après séparation de la phase aqueuse et de la phase organique constituée par le lactame, cette dernière phase organique étant, de préférence recyclée dans le milieu alimenté dans la colonne de déshydratation ou recyclée dans la seconde colonne, ou encore soumise à un autre traitement tel que l'évaporation en couche mince ou cristallisation,
- soit, une évaporation de lactame réalisée dans un évaporateur à film ou couche mince,
- ou, une cristallisation du lactame dans l'eau, une solution aqueuse de lactame ou du lactame.

Ces traitements peuvent être combinés, notamment le lavage à l'eau peut être réalisé préalablement à l'évaporation ou la cristallisation.

Selon une caractéristique de l'invention, le lactame est avantageusement l'ε-caprolactame obtenu par hydrolyse cyclisante d'aminocapronitrile soit en phase vapeur soit en phase liquide avec ou sans solvant.

L'aminonitrile est, par exemple, produit par hydrogénation partielle de l'adiponitrile. Cette réaction est notamment décrite dans les brevets US 4,601,859, US 2,762,835, US 2,208,598, DE 4,235,466, US 5,981,790.

L'ammoniac contenu dans le milieu réactionnel d'hydrolyse cyclisante est éliminé par distillation ou flashage.

Le milieu réactionnel alimenté dans le procédé de l'invention peut comprendre au moins 99,9 % en poids de lactame. Ce domaine de concentration n'est donné qu'à titre indicatif. Ainsi, des milieux contenant moins de lactame peuvent être également purifiés par le procédé de l'invention.

A titre d'indication non limitatives, les conditions de fonctionnement des différentes colonnes du second mode de réalisation de l'installation selon l'invention sont données ci-après.

La première étape de déshydratation est mise en oeuvre à une température comprise entre 100°C et 130°C environ, à une pression comprise entre 50 mbar et 200 mbar environ.

La fraction déshydratée est alimentée dans une deuxième colonne de distillation dans laquelle la deuxième étape de séparation des produits à points d'ébullition élevés est réalisée par production d'un pied de distillation.

Les conditions de fonctionnement de cette colonne sont une température de pied de colonne inférieure à 150°C et une pression comprise entre 5 et 10 mbar.

La fraction de tête ainsi produite comprend moins de 100 ppm d'impuretés à point d'ébullition plus élevé que le lactame

Cette fraction de tête est alimentée dans une nouvelle colonne de distillation dont les conditions de fonctionnement sont une température de pied de colonne inférieure à 150°C et une pression comprise entre 5 et 10 mbar.

Le lactame, notamment l'ε-caprolactame produit comme fraction intermédiaire présente un degré de pureté satisfaisant les critères requis pour la fabrication de polyamide, notamment pour les applications textiles. De préférence, cette fraction intermédiaire est soutirée en phase vapeur de la colonne pour obtenir une concentration très faible en produits de point d'ébullition élevé. Toutefois, il est également possible de soutirer cette fraction intermédiaire en phase liquide, sans sortir du cadre de l'invention.

Le pied de distillation obtenu en pied de colonne est recyclé, dans un mode de réalisation de l'invention, dans la seconde colonne de distillation.

Dans un mode de réalisation préféré de l'invention et conformément au procédé de purification du lactame de l'invention, le pied de distillation recueilli en pied de seconde colonne est alimenté dans une étape de séparation du lactame, par exemple un procédé d'évaporation en film ou couche mince à une température inférieure à 150°C et sous une pression comprise entre 5 et 10 mbar.

Dans un autre mode de réalisation préféré de l'invention, ce pied de distillation est soumis à un procédé de cristallisation pour récupérer le lactame, plus particulièrement le caprolactame.

Il est également possible de traiter ce pied de distillation par de l'eau pour extraire les sels, plus particulièrement les sulfates provenant de la neutralisation des bases libres présentes dans le milieu d'hydrolyse. La phase organique récupérée contenant le lactame est soit recyclée dans la colonne de déshydratation ou la seconde colonne, soit soumise à une nouvelle étape de séparation du lactame telle qu'une évaporation en film ou couche mince ou une cristallisation.

Selon l'invention, une base choisie dans le groupe comprenant les hydroxydes alcalins, les hydroxydes alcalino-terreux, les carbonates métalliques par exemple l'hydroxyde de sodium, l'hydroxyde de lithium, l'hydroxyde de potassium, l'hydroxyde de magnésium, l'hydroxyde de calcium, les carbonates de sodium, magnésium, lithium, les carbonates mixtes peuvent être utilisés comme composé basique. De préférence, une solution aqueuse d'hydroxyde de sodium est utilisée. La quantité de base ajoutée peut varier dans de larges limites. Avantageusement, elle est comprise entre 0,05 et 2 g de base pour 1 kg de caprolactame ou lactame.

Comme indiqué précédemment cette base est ajoutée soit dans le milieu à traiter alimenté dans l'étape de déshydratation soit dans la fraction de tête issue de la seconde colonne du second mode de réalisation de l'installation, avant son alimentation dans la troisième colonne.

Selon l'invention, il est également possible d'ajouter dans le milieu alimenté dans l'étape de déshydratation, un acide de préférence l'acide sulfurique. La quantité d'acide ajoutée est déterminée en fonction de la quantité de bases libres présentes dans le milieu à traiter. Cette quantité de bases libres est déterminée par dosage potentiométrique du milieu. La quantité d'acide ajoutée est comprise entre 0,5 mole d'acide par mole de bases libres et une mole d'acide par mole de bases libres, de préférence entre 0,7 et 0,9 mole d'acide par mole de bases libres. Ce domaine de concentration permet d'obtenir des sels fusibles et une action corrosive faible du milieu.

D'autres avantages, détails de l'invention apparaîtront plus clairement au vu de la description détaillée d'exemples faite en référence aux figures annexées dans lesquelles:

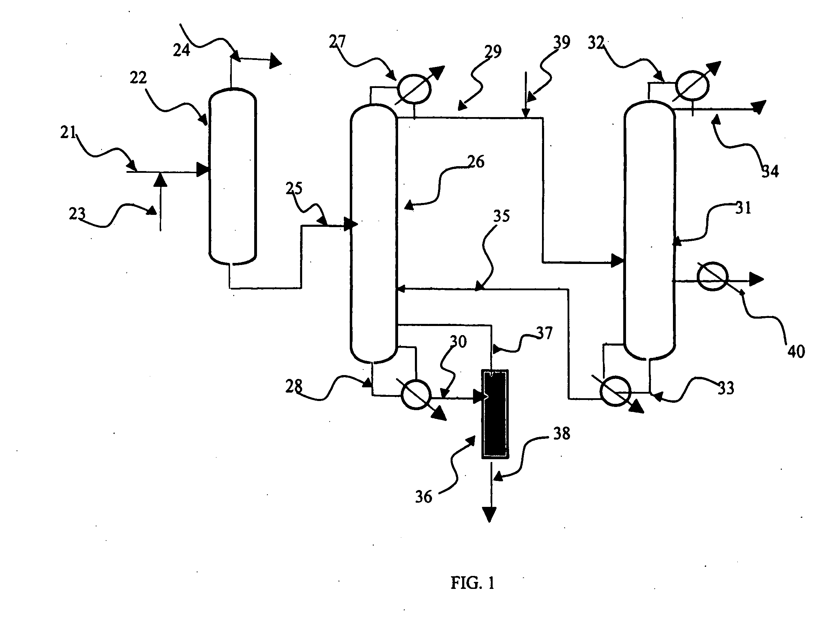

- la figure 1 représente un schéma synoptique d'un mode de réalisation de l'installation de l'invention

### Exemple 1

Un milieu d'hydrolyse d'aminocapronitrile après distillation (stripping) de l'ammoniac est traité dans une installation conforme à l'invention et représentée schématiquement par la figure 1, selon un mode de réalisation du procédé de purification du caprolactame.

Les caractéristiques de ce milieu sont les suivantes :
Indice permanganate (Ik_{Mno₄}) : 381
Teneur en bases volatiles (I_{Bv}) : 232 meq/kg
Indice UV (lᵤᵥ): 12
De plus, la concentration en bases libres déterminée par dosage potentiométrique est égale à 189 meq par kg de milieu ou solution.
En se référant à la figure 1, ce milieu est conformément à l'invention, alimenté par le conduit 21 dans la première colonne 22 de distillation, après addition par le conduit 23, de 0,8 mole d'acide sulfurique sous forme d'une solution concentrée par mole de bases libres contenues dans le milieu à traiter.

La colonne 22 a une température de pied inférieur à 130°C et une pression de 70 mbar.
La fraction de tête 24 est constituée par de l'eau.
Le pied de distillation 25 contient une quantité en eau inférieure à 0,5 % en poids.
Ce pied de distillation 25 est alimenté dans une deuxième colonne 26 travaillant sous reflux 27, 28.
La fraction de tête 29, issue de cette deuxième colonne 26 constituée essentiellement par du caprolactame et de composés de point d'ébullition plus faible que celui du caprolactame, est alimentée dans une troisième colonne 31 travaillant également sous reflux 32, 33, après addition, par le conduit 39 dans cette fraction de tête de 0,6 g de soude pure sous forme de solution concentrée par kg de caprolactame.
La fraction de tête 34 de la troisième colonne 31 constituée par des composés à point d'ébullition plus bas que celui du caprolactame est éliminée.
Le pied de distillation 35 est recyclé, dans le mode de réalisation illustré, dans la seconde colonne 26.
Le caprolactame purifié est soutiré de cette troisième colonne 31 sous forme d'une fraction intermédiaire 40. Ce soutirage est réalisé soit sous forme gazeuse à la partie inférieure de la colonne, soit sous forme liquide dans la partie supérieure de la colonne. Les caractéristiques du caprolactame produit sont :
Indice permanganate (lk_{Mno₄}): 2
Teneur en bases volatiles (I_{Bv}): 0,25 meq/kg
Indice UV (lᵤᵥ): 0,03
Selon le procédé de l'invention, le pied de distillation 30 de la seconde colonne 26 constitué majoritairement par du caprolactame et comprenant comme impuretés des composés à point d'ébullition plus élevé que celui du caprolactame et des sels formés par la neutralisation des bases libres par l'acide sulfurique est alimenté dans un évaporateur 36 à film raclé fonctionnant sous une pression de 10 mbar et une température de 150°C. Le caprolactame évaporé est recyclé, dans le mode de réalisation illustré, par le conduit 37 dans la deuxième colonne 26.
Les produits non évaporés sont éliminés par le conduit 38.
Le bilan pondéral du caprolactame alimenté dans la première colonne 22 et celui produit sous forme de fraction intermédiaire 40 de la troisième colonne 31 montre que 98,9 % du caprolactame engagé est récupéré avec un degré de pureté élevé et convenable

### Exemple 2

L'exemple 1 est répété à la différence que le pied de distillation 30 de la seconde colonne est traité par addition d'eau pour obtenir une solution à environ 80 % en poids de caprolactame. Le mélange est soumis à une agitation. Après mélange, deux phases liquides se forment. La phase inférieure est une phase aqueuse qui comprend 60 % en poids de sulfates d'amines, correspondant à environ 70 % de la quantité d'amines présentes dans le caprolactame engagé dans le procédé de purification, à la colonne de déshydratation. La phase supérieure essentiellement constituée de caprolactame est recyclée dans la première colonne de distillation.

Par ce procédé, 99 % du caprolactame engagé dans la colonne de distillation est récupéré à un degré de pureté élevé, convenable pour une utilisation de ce produit dans la fabrication de polyamide, notamment pour les applications textiles.

### Exemple 3

L'exemple 1 est répété à la différence que le pied de distillation 30 de la seconde colonne est traité par un procédé de cristallisation en une étape du caprolactame
le pied de distillation est concentré sous pression réduite pour obtenir une concentration pondérale en eau d'environ 8%.

La solution concentrée est à une température de 40°C puis refroidie jusqu'à une température voisine de 20°C avec une vitesse de refroidissement d'environ 10°C par heure.

Les cristaux formés sont récupérés par filtration sur un fritté. Les eaux mères récupérées sont stockées pour un éventuel recyclage.
Le gâteau solide de cristaux de caprolactame est lavé par une solution aqueuse saturée en caprolactame. Le taux de lavage en base humide est avantageusement compris entre 1 et 5.

Le caprolactame récupéré a une pureté suffisante pour être recyclé dans le procédé de distillation.

Par ce procédé, 99 % du caprolactame engagé dans la colonne de distillation est récupéré à un degré de pureté élevé, convenable pour une utilisation de ce produit dans la fabrication de polyamide, notamment pour les applications textiles.

## Revendications

1. Procédé de purification de lactames obtenus par hydrolyse cyclisante d'un aminonitrile, consistant à éliminer du milieu réactionnel d'hydrolyse, l'ammoniac et éventuellement le solvant, **caractérisé en ce :**
• **qu'**il consiste à éliminer l'eau dans une première (22) colonne de distillation, à distiller le lactame dans une seconde (26) colonne de distillation pour séparer les composés moins volatils que le lactame et récupérer le lactame comme fraction de tête et à alimenter ladite fraction de tête dans une troisième colonne (31) de distillation pour récupérer les composés plus volatils que le lactame en tête de colonne et le lactame purifié comme fraction intermédiaire,
• **qu'** un composé basique est ajouté au lactame
• **que** le pied de distillation (28) de la seconde colonne (26) de distillation est soumis à un traitement pour récupérer le lactame contenu et recycler ce lactame récupéré dans le procédé de purification du lactame, le traitement consistant à séparer le lactame par un procédé choisi dans le groupe comprenant une évaporation en film ou couche mince, une cristallisation et/ou un lavage à l'eau.

2. Procédé selon la revendication 1, **caractérisé en ce que** le composé basique est ajouté au lactame avant alimentation dans la première colonne (22) de déshydratation.

3. Procédé selon l'une des revendications 1 à 2, **caractérisée en ce que** le composé basique est choisi parmi les hydroxydes alcalins.

4. Procédé selon l'une des revendications précédentes **caractérisé en ce que** la quantité de composés basiques ajoutés est comprise entre 0,05g et 2g de composés basiques pour 1 kg de lactame.

5. Procédé selon l'une des revendications précédentes, **caractérisé en ce que** le milieu comprenant le lactame à purifier est obtenu par traitement sur résine échangeuse d'ions du milieu réactionnel d'hydrolyse d'aminonitrile, après élimination d'ammoniac.

6. Procédé selon l'une des revendications précédentes **caractérisé en ce que** le lactame est le caprolactame.

7. Procédé selon l'une des revendications précédentes, **caractérisé en ce que** le milieu réactionnel d'hydrolyse est soumis à une hydrogénation avant élimination de l'ammoniac.

8. Procédé selon l'une des revendications précédentes, **caractérisé en ce que** le milieu réactionnel d'hydrolyse est soumis à une hydrogénation après élimination de l'ammoniac.

9. Procédé selon l'une des revendications précédentes, **caractérisé en ce que** l'hydrolyse cyclisante est mise en oeuvre en phase vapeur ou en phase liquide.

10. Procédé selon l'une des revendications précédentes, **caractérisé en ce que** l'hydrolyse cyclisante est mise en oeuvre en phase liquide

## Patentansprüche

1. Verfahren zur Aufreinigung von Lactamen, die durch cyclisierende Hydrolyse eines Aminonitrils erhalten wurden, das darin besteht, dass man das Ammoniak und gegebenenfalls das Lösungsmittel aus dem Hydrolysereaktionsmedium entfernt, **dadurch gekennzeichnet, dass**
• es darin besteht, in einer ersten Destillationskolonne (22) das Wasser zu entfernen, in einer zweiten Destillationskolonne (26) das Lactam zu destillieren, um die Verbindungen, die weniger flüchtig als das Lactam sind, abzutrennen und das Lactam als Kopffraktion zu gewinnen und diese Kopffraktion einer dritten Destillationskolonne (31) einzuspeisen, um die Verbindungen, die stärker flüchtig als das Lactam sind, am Kolonnenkopf und das aufgereinigte Lactam als Zwischenprodukt zu gewinnen,
• man das Lactam mit einer basischen Verbindung versetzt,
• man den Destillationssumpf (28) der zweiten Destillationskolonne (26) einer Behandlung unterzieht, um das enthaltene Lactam zu gewinnen und das erhaltene Lactam in das Lactamaufreinigungsverfahren zurückzuführen, wobei die Behandlung daraus besteht, dass man das Lactam durch ein Verfahren, ausgewählt aus der Gruppe umfassend eine Filmschichtverdampfung oder Dünnschichtverdampfung, eine Kristallisation und/oder ein Waschen mit Wasser, abtrennt.

2. Verfahren nach Anspruch 1, **dadurch gekennzeichnet, dass** man das Lactam vor dem Einspeisen in die erste Entwässerungskolonne (22) mit der basischen Verbindung versetzt.

3. Verfahren nach einem der Ansprüche 1 bis 2, **dadurch gekennzeichnet, dass** die basische Verbindung aus der Reihe der Alkalihydroxide stammt.

4. Verfahren nach einem der vorhergehenden Ansprüche, **dadurch gekennzeichnet, dass** die Menge der zugegebenen basischen Verbindungen zwischen 0,05 g und 2 g basische Verbindungen pro 1 kg Lactam liegt.

5. Verfahren nach einem der.vorhergehenden Ansprüche, **dadurch gekennzeichnet, dass** das Medium, das das aufzureinigende Lactam enthält, durch Behandeln des Reaktionsmediums der Aminonitrilhydrolyse an Ionenaustauscherharz nach dem Entfernen von Ammoniak erhalten wird.

6. Verfahren nach einem der vorhergehenden Ansprüche, **dadurch gekennzeichnet, dass** es sich bei dem Lactam um Caprolactam handelt.

7. Verfahren nach einem der vorhergehenden Ansprüche, **dadurch gekennzeichnet, dass** das Hydrolysereaktionsmedium vor dem Entfernen des Ammoniaks hydriert wird.

8. Verfahren nach einem der vorhergehenden Ansprüche, **dadurch gekennzeichnet, dass** das Hydrolysereaktionsmedium nach dem Entfernen des Ammoniaks hydriert wird.

9. Verfahren nach einem der vorhergehenden Ansprüche, **dadurch gekennzeichnet, dass** die cyclisierende Hydrolyse in der Dampfphase oder in flüssiger Phase erfolgt.

10. Verfahren nach einem der vorhergehenden Ansprüche, **dadurch gekennzeichnet, dass** die cyclisierende Hydrolyse in flüssiger Phase erfolgt.

## Claims

1. Process for the purification of lactams obtained by the cyclizing hydrolysis of an aminonitrile, consisting in removing, from the hydrolysis reaction medium, the ammonia and optionally the solvent, **characterized in that**:
• it consists in removing the water in a first distillation column (22), in distilling the lactam in a second distillation column (26), in order to separate the compounds which are less volatile than the lactam and to recover the lactam as top fraction, and in feeding said top fraction to a third distillation column (31), in order to recover the compounds which are more volatile than the lactam at the top of the column and the purified lactam as intermediate fraction,
• a basic compound is added to the lactam,
• the distillation bottom product (28) from the second distillation column (26) is subjected to a treatment in order to recover the lactam present and to recycle this recovered lactam in the process for the purification of the lactam, the treatment consisting in separating the lactam by a process chosen from the group consisting of a thin-film or thin-layer evaporation, a crystallization and/or a washing with water.

2. Process according to Claim 1, **characterized in that** the basic compound is added to the lactam before feeding to the first dehydration column (22) .

3. Process according to either of Claims 1 and 2, **characterized in that** the basic compound is chosen from alkali metal hydroxides.

4. Process according to one of the preceding claims, **characterized in that** the amount of basic compounds which are added is between 0.05 g and 2 g of basic compounds per 1 kg of lactam.

5. Process according to one of the preceding claims, **characterized in that** the medium comprising the lactam to be purified is obtained by treatment through an ion-exchange resin of the aminonitrile hydrolysis reaction medium, after removal of ammonia.

6. Process according to one of the preceding claims, **characterized in that** the lactam is caprolactam.

7. Process according to one of the preceding claims, **characterized in that** the hydrolysis reaction medium is subjected to a hydrogenation, before removal of the ammonia.

8. Process according to one of the preceding claims, **characterized in that** the hydrolysis reaction medium is subjected to a hydrogenation, after removal of the ammonia.

9. Process according to one of the preceding claims, **characterized in that** the cyclizing hydrolysis is carried out in the vapor phase or in the liquid phase.

10. Process according to one of the preceding claims, **characterized in that** the cyclizing hydrolysis is carried out in the liquid phase.
